# EUROPEAN PATENT SPECIFICATION

(11) **EP 3 557 235 B1**
(45) Date of publication and mention of the grant of the patent: **07.09.2022**
(21) Application number: 19169166.6
(22) Date of filing: 15.04.2019
(51) Int. Cl.: B08B 9/032, B08B 9/46

(54) **A METHOD FOR MEASURING AN ENTITY OF INTEREST IN A STREAM OF RINSING WATER**
VERFAHREN ZUM MESSEN EINER EINHEIT VON INTERESSE IN EINEM SPÜLWASSERSTROM
PROCÉDÉ DE MESURE D'UNE ENTITÉ D'INTÉRÊT DANS UN FLUX D'EAU DE RINÇAGE

(30) Priority: 19.04.2018 DK PA201870235
(43) Date of publication of application: 23.10.2019
(73) Proprietor: Purgatio A/S, 4070 Kirke Hyllinge (DK)
(72) Inventor: PETERSEN, Michael Egede, 4300 Holbaek (DK)
(74) Representative: Nordic Patent Service A/S

(56) References cited:
- EP-A1- 0 903 320
- CN-A- 103 245 765
- DE-A1-102008 056 522
- JP-A- 2000 334 402

## Description

### TECHNICAL FIELD

The disclosure relates to a method and plant for measuring an entity of interest in a liquid stream, particularly an entity of interest in a stream of rinsing water, primarily in the context of clean-in-place (CIP) cleaning of containers used in pharmaceutical, food, beverage, or similar industries.

### BACKGROUND

Various clean-in-place (CIP) processes for cleaning containers in pharmaceutical, food, beverage, or other industries are known from the prior art.

For example, US20060042665A1 discloses a method of cleaning equipment using CIP procedures and a pre-treatment solution prior to the conventional CIP cleaning process. The pre-treatment step improves the degree of softening of the soil, and thus facilitates its removal.

EP0903320A1 relates to a plant for cleaning a filling plant having a filler and conveyor, which plant comprises a CIP cleaning system for the filler and the conveyor as well as a filler external cleaning system and a hot water flooding system.

It is known that air bubbles present in the cleaning liquid may negatively affect the cleaning effectiveness and the quality of cleaning. For example, in ultrasonic cleaning, it is known that the sound pressure of ultrasonic waves is greatly affected by the concentration of dissolved air contained in the cleaning liquid. The transmission of sound waves is impeded, energy loss is caused, the sound pressure is reduced, and the cleaning power is reduced. Therefore, conventionally, a degassing device is attached to the ultrasonic cleaning device to reduce the concentration of dissolved air in the cleaning solution, so that efficient and stable cleaning can be realized.

JP2008296217A relates to the above problem and discloses a degassing apparatus for removing dissolved air in a cleaning solution and an ultrasonic cleaning apparatus using the degassing apparatus.

In the pharmaceutical, food, beverage, or other industries requiring high levels of hygiene, the internal cleaning of tanks and containers is essential. Generally, there is a need for monitoring the cleaning process, thus allowing for determining e.g. the state of the cleaning process, the cleaning effectiveness, whether the cleaning meets certain sanitary requirements, etc.

US20170282225A1 discloses an invention, wherein the uniform distribution of the cleaning liquid and its degree of contamination are monitored by calorimetric flow measurement.

For this purpose, a heating element is disposed in the flow-off area of the cleaning liquid.

US7682460 provides a method for cleaning chemical process and hydrocarbon processing apparatuses, wherein the cleaning method is monitored by performing analyses such as e.g. viscosity and density analysis on the circulating hydrocarbon-based fluid.

EP1882914A2 discloses a method that involves arranging a measuring point, which represents a measuring position, at a wall surface of a tank, a container, or a pipe. A temporally stepwise or continuous varying signal (S(t)) for presence of a medium or media composite characteristics is received and evaluated at the measuring point. Molecules of the medium arrived into a region of the measuring point are detected by a sensor. Temporal variation of the signal (dS/dt) or rate of change of temporal variation (dS/dt2) of alternating electrical field is detected as a characteristic signal for evaluating the signal.

Finally, US2004118432A1 relates to a monitoring device and monitoring methods that optimize the control sequence of the inlet valves and the outlet valves of the fluid storage tanks and the waste disposal lines of a clean-in-place system.

It is an object of the present invention to provide a method and plant that allows for precise and reproducible measurement of an entity of interest in a stream of rinsing water in a CIP cleaning system. The entity of interest could be e.g. the concentration of a compound or contaminant. The method and plant can be used e.g. for monitoring a cleaning process and for validation of washing effectiveness of same cleaning process. Validation of washing effectiveness is of particular interest when cleaning containers, tanks, pipes, or other equipment that must meet cleanliness requirements, as is often the case in pharmaceutical, food, beverage, or similar industries. Said object of the invention is achieved by the features of the independent claims. Further implementation forms are apparent from the dependent claims, the summary, the description, and the figures.

### SUMMARY

According to a first aspect, the invention provides a method for measuring an entity of interest in a stream of rinsing water, comprising
a. supplying a stream of water to a unit previously subjected to a washing process for rinsing at least a part of the surfaces of the washed unit,
b. supplying a stream of air to the washed unit,
c. collecting a mixture of water and air at an outlet of the unit,
d. deaerating the mixture of water and air using a cyclone to produce an aqueous stream, and
e. measuring the concentration of an entity of interest in the aqueous stream.

The inventor of the present invention has realized that air bubbles in the rinsing water may cause problems by influencing measurements, rendering measurement imprecise and unreproducible. Step d of the present invention removes air bubbles that might otherwise influence measurement of the aqueous stream in step e.

To obtain a uniform cleaning of the unit, a stream of air is supplied concurrently with the stream of water during rinse. The stream of air is used to prepare a pressure above the atmospheric pressure to propel emptying of the unit and to avoid build-up of a standing water phase inside the washed unit, also referred to as the CIP client, during rinse.

The method according to the invention may find use in various applications, incl. cleaning of e.g. bottles for beverages, reaction chambers for chemicals, containers for wastewater, etc.

In a possible implementation form of the first aspect, the method further comprises the steps of
f. comparing the measured concentration of the entity of interest with a predetermined concentration and
g. determining that the washing process is validated if the measured concentration is at or below the predetermined concentration.

The effect of steps f and g is to assess and validate the effectiveness of the washing process.

The measurement of the entity of interest may be performed as a single occurrence, or the measurement may be repeated as required by the circumstances. In a possible implementation of the invention, the steps a to f are repeated if the measured concentration is above a predetermined concentration.

The effect hereof is to control the washing process and to ensure that the wash is thorough and that the washed unit meets specified requirements if necessary.

The deaeration may be performed batchwise or continuously as required by the circumstances. When performed batchwise, the deaeration is preferably performed immediately prior to the measuring of the entity of interest to avoid air bubbles in the measured sample. In a possible implementation form of the invention, the mixture of water and air is continuously deaerated to allow for continuous or discrete measuring of the entity of interest.

The effect hereof is to continuously remove air bubbles that might otherwise influence a later measuring step and, thus, to generate a deaerated stream of rinsing water that can be subjected to measurement.

While it may be useful in certain embodiments of the invention to perform discrete measurements, it may often be preferred that the concentration of the entity of interest is measured continuously. The effect hereof is to obtain continuous measurement data on the deaerated rinsing water, allowing for immediate intervention when the concentration of the entity of interest is at, below and/or above a predetermined concentration. Such continuous monitoring may be advantageous e.g. in pharmaceutical production to ensure e.g. patient security.

When discrete measurements are performed, analysis may take a certain amount of time before the result is available. Hence, this type of measurement will result in a certain lag in response time. It may therefore often be preferred that the measuring of the concentration of an entity of interest in the aqueous stream is performed continuously as an in-line or on-line process.

The entity of interest may be selected among a plurality of possibilities. In a certain implementation form of the invention, the entity of interest is one or more of Total Organic Carbon (TOC), O₂, CO₂, pH, bioburden, or conductivity. The effect hereof is to determine composition, contamination, chemical properties, and/or physical properties of the deaerated rinsing water, thereby assessing the state of the washed unit and monitoring the washing process.

According to a second aspect, the invention provides a plant for measuring an entity of interest in a stream of rinsing water, as defined in claim 8.

The plant allows for the implementation of possible embodiments of the method according to the invention, in which a previously washed unit can be rinsed without build-up of any standing water phase inside the unit during rinse and in which the rinsing water is deaerated prior to measurement, thus removing air bubbles that might otherwise influence measurement. Inlets may e.g. be in the form of jets, nozzles, sprinklers, spray heads, etc.

In order to infer and assess the effectiveness of the washing process and thereby the state of cleanliness of the container, measurements are compared to predetermined and/or predefined standards, e.g. predetermined concentrations. While the comparison may be carried out or supervised by a human, having a computer software to perform the task in an automated process may often be preferred.

In a possible implementation form of the second aspect, the plant comprises a computer software program capable of comparing the measured concentration of the entity of interest with a predetermined concentration of the entity of interest and determining that the washing process is validated if the measured concentration is at or below the predetermined concentration.

The effect hereof is that the washing effectiveness and the cleanliness control and validation are performed in the most efficient manner, thus saving time and manpower.

Compounds requiring marketing approval by an authority such as e.g. a food or drug authority need to fulfill certain requirements with regards to e.g. reproducibility of the preparation process. Meeting such requirements requires a reproducible cleaning process for cleaning of equipment used e.g. in handling, preparation, storage, processing, etc. of the compounds. The plant according to the invention is capable of accommodating the requirements of any authority, notably an authority for granting marketing approval for pharmaceutical products.

The effect hereof is that the present invention can be used to wash and validate the washing of a container used e.g. in handling, preparation, storage, processing, etc. of a compound requiring marketing approval by an authority.

The rinsing water is collected, preferably continuously, at the outlet of the washed container and without build-up of any standing water phase in the washed container. Furthermore, the rinsing water is supplied, preferably continuously, to the cyclone for deaeration prior to measurement. While the rinsing water may flow naturally from the unit to the cyclone, flow or transport of the rinsing water may also be achieved e.g. by overpressure in the washed container and/or by using a pumping system.

In a possible implementation form of the second aspect, a pump is used for transporting the collected mixture of water and air from the outlet of the unit to the cyclone. The effect hereof is to supply a sufficient amount of the rinsing water to the cyclone to avoid the build-up of a standing water phase.

In a possible implementation form of the second aspect, the pump is a side channels pump or water ring pump. The effect hereof is to ensure the most effective transport of the mixture of the rinsing water and the air bubbles.

Deaeration of the rinsing water prior to measurement may be achieved by various means and apparatuses, incl. e.g. by sonication and stirring under reduced pressure (vacuum degasification), gas-liquid separation membranes, thermal regulation, etc. In a possible implementation form of the second aspect, the cyclone is a hydrocyclone. The effect hereof is to efficiently produce a deaerated aqueous stream that can be subjected to measurement, using an in-line operation.

For measurement of the entity of interest in the aqueous stream, a range of different measuring systems, sensors, and/or combinations thereof may be used. In a possible implementation form of the second aspect, the sensor is one or more of pH electrode, TOC sensor, bioburden sensor, oxygen sensor, CO₂ sensor, or conductivity sensor. The effect hereof is to ensure proper measurement of the entity of interest. The method and plant of the present invention may find use in various applications and contexts in which measurement of an entity of interest in a liquid stream, particularly in an aqueous stream, is desired. In a possible implementation form of the first aspect and its implementation forms, the invention comprises validating a washing process of a unit previously used for accommodating a compound requiring marketing approval by an authority.

The effect hereof is to provide a method and plant that can be used e.g. in the handling, preparation, storage, processing, etc. of a compound requiring marketing approval by an authority.

These and other aspects will be apparent from and the embodiment(s) described below.

### BRIEF DESCRIPTION OF THE DRAWINGS

In the following detailed portion of the present disclosure, the aspects, embodiments, and implementations will be explained in more detail with reference to the example embodiments shown in the drawings, in which:

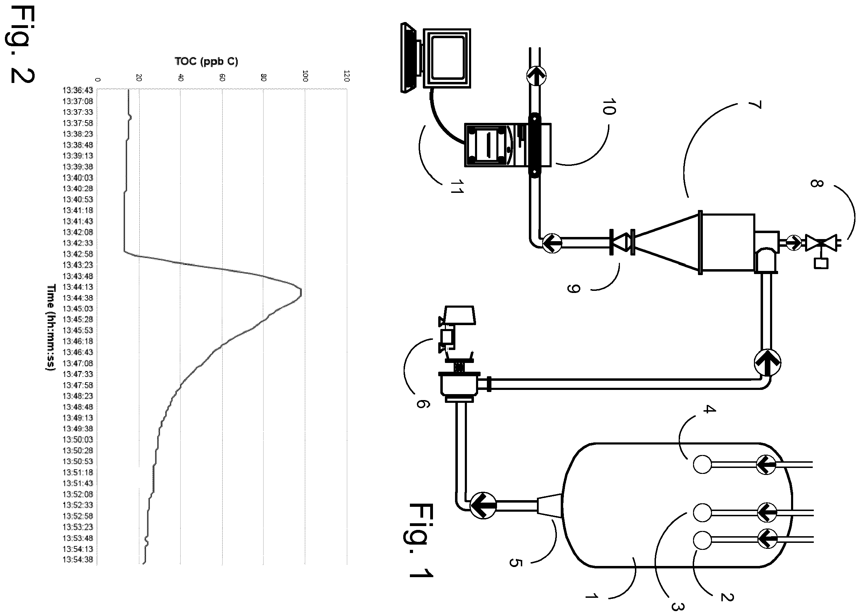

Fig. 1 is a simplified scheme of a possible embodiment of the present invention, showing a clean-in-place (CIP) plant with an integrated cyclone for deaerating rinsing water and with an integrated sensor for performing measurements on the deaerated rinsing water.
Fig. 2 is a plot with measurement results obtained in a possible embodiment of the present invention. The plot shows measured total organic carbon (TOC) data vs. time, as collected online and continuously in a stream of deaerated rinsing water in a CIP plant with an integrated hydrocyclone for deaerating the rinsing water and with an integrated sensor.

### DETAILED DESCRIPTION

Fig. 1 shows a simplified scheme of a possible embodiment of the plant of the present invention. The plant comprises a container 1 to be washed.

The container 1 is of a kind used in pharmaceutical, food, beverage, or similar industries. The container 1 may be, but does not have to be, made of stainless steel.

Prior to washing, the container 1 has been used for containing a liquid, e.g. water, with e.g. a product, compound, mixture of compounds, or an active ingredient dissolved or otherwise contained therein. Possible products are pharmaceutical products such as e.g. insulin, protein, polypeptide, or a drug, dairy products such as e.g. cheese, whey, milk, or sour milk, or food or beverage products.

The container 1 is a sealed container. In the embodiment of Fig. 1, the container 1 has an inlet 2 for water and an inlet 3 for air. The inlets 2 & 3 are used for supplying water and air during rinse. In an alternative embodiment, water and air may be supplied through one and the same inlet. An additional inlet 4 for supplying cleaning fluids during wash is also shown in Fig. 1. Further additional inlets may be present in alternative embodiments of the invention.

A possible washing procedure for washing the inner of the container could comprise the steps 1) pre-rinse to drain, 2) base wash with recirculation using e.g. 2-5 % sodium hydroxide (NaOH) at elevated temperature (e.g. 80 °C), 3) inter-rinse to drain, 4) acid wash with e.g. cold nitric acid (HNO₃) for neutralizing the NaOH of the base wash (note that due to its oxidizing nature, nitric acid promotes the resistance of stainless steel to corrosion), 5) inter-rinse to drain, and 6) final rinse.

During rinse, inner surfaces of the container 1 are rinsed with water and air. The water and air streams are supplied concurrently. Possibly, the air is in the form of compressed air. The air stream functions to ensure a uniform rinse and prevents build-up of a standing water phase inside the container. The ratio between water and air is such that the air helps propelling the water out of the container 1. This is accomplished when using roughly an amount of air that is two-to-three times the amount of water. The mixture of water and air, also referred to as the rinsing water, exits the container 1 through an outlet 5.

After exiting the container 1, the rinsing water enters into a connected hydrocyclone 7 via a pump 6. The function of the pump 6 is to assist or enable transport of the collected rinsing water from the outlet 5 to the hydrocyclone 7. The pump 6 should be suited for container emptying and CIP return applications where air and gas is entrained in the pumped liquid. The pump 6 can be of type side channel pump or water ring pump. Examples of applicable pumps are CSA series pumps (closed coupled sanitary centrifugal pumps with open impeller and independent shaft support) and ASH series pumps (close coupled sanitary self-priming pumps with independent shaft support), as produced by CSF Inox S.p.A., or the Alfa Laval LKH Prime UltraPure pump. In case the container 1 is e.g. a pressurized container with sufficiently high gauge pressure (e.g. of 0.5-0.2 bar), no pump may be needed to transport the rinsing water from the container 1 to the hydrocyclone 7. In a pressureless container, e.g. in a container that is open to the surroundings, a pump will generally be needed in order to pump out the mixture of water and air from the container.

In the hydrocyclone 7, the rinsing water is separated into a predominantly gas phase (air) and a predominantly aqueous phase (water), the latter also referred to as aqueous stream. That is, the hydrocyclone 7 functions to continuously deaerate the rinsing water collected from the container 1, thereby producing a deaerated aqueous stream that can be readily subjected to online measurement and analysis by a sensor without signal noise or other disturbances caused by the presence of air bubbles. In principle, any hydrocyclone capable of such water/gas separation can be used. The separated air exits through an outlet 8, typically in the form of a vent, at the top of the hydrocyclone while the separated aqueous stream exits through an outlet 9 at the bottom of the hydrocyclone.

The outlet at the bottom of the hydrocyclone 7 is connected to a sensor 10 to which the aqueous stream is exposed for measuring a specified property, e.g. conductivity, Total Organic Carbon (TOC), pH, and/or bioburden. Measurements on the aqueous stream are carried out online and/or inline and continuously by the sensor 10 in e.g. a flow cell. An example of an applicable sensor could be the Thornton 5000TOCi sensor with M800 multi-parameter transmitter that uses ultraviolet (UV) oxidation with differential conductivity to determine TOC concentrations.

The sensor 10 is connected to a controlling software 11 that monitors the measurement. The software 11 is capable of comparing measured values with predetermined or pre-defined values, determining if a measured value, e.g. a measured concentration value, is below, at, or above the predetermined value, thereby accessing e.g. the degree of cleanliness of the container 1 and, ultimately, validating the overall washing process if the measured value is at or below the predetermined value. After measurement, the aqueous stream may be recirculated back into the container 1, optionally after being supplemented with fresh water, or it may be discarded to drain.

Fig. 2 shows a plot with measurement results obtained in a possible embodiment of the present invention. Total organic carbon (TOC) was measured vs. time online and continuously in a stream of deaerated rinsing water in a CIP plant with an integrated hydrocyclone for deaerating the rinsing water prior to measurement and with an integrated sensor. In the embodiment used in Fig. 2, measurement was performed on a minor partial stream of rinsing water diverted from the main stream of rinsing water. The minor partial stream of rinsing water was sent directly to drain after measurement.

At time ca. 13:41:00, common table sugar (sucrose) corresponding to an amount of 20 sugar granules was added to the container of the plant, resulting in a rapid increase in the total organic carbon content from 14 ppb to 98 ppb. Following recirculation with 400 L of clean water, the organic carbon content dropped to 23 ppb. That is, the 20 granules of table sugar raised the TOC concentration from 14 ppb to 23 ppb.

Deaeration of the rinsing water prior to measurement removed air bubbles from the rinsing water, allowing for the measurements shown in Fig. 2.

The various aspects and implementations have been described in conjunction with various embodiments herein. However, other variations to the disclosed embodiments can be understood and effected by those skilled in the art in practicing the claimed subject-matter, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems.

The reference signs used in the claims shall not be construed as limiting the scope.

## Claims

1. A method for measuring an entity of interest in a stream of rinsing water, comprising
a. supplying a stream of water to a unit (1) previously subjected to a washing process for rinsing at least a part of the surfaces of the washed unit (1),
b. supplying a stream of air to the washed unit (1),
c. collecting a mixture of water and air at an outlet (5) of the unit (1),
**characterized in that** said method comprises
d. deaerating the mixture of water and air using a cyclone (7) to produce an aqueous stream, and
e. measuring the concentration of an entity of interest in the aqueous stream.

2. The method according to claim 1, further comprising the steps of
f. comparing the measured concentration of the entity of interest with a predetermined concentration and
g. determining that the washing process is validated if the measured concentration is at or below the predetermined concentration.

3. The method according to claim 2, wherein the steps a to f are repeated if the measured concentration is above the predetermined concentration.

4. The method according to any one of the claims 1 to 3, wherein the mixture of water and air is continuously deaerated.

5. The method according to any one of the claims 1 to 4, wherein the concentration of the entity of interest is measured continuously.

6. The method according to any one of the claims 1 to 5, wherein the measuring of the concentration of an entity of interest in the aqueous stream is performed continuously as an in-line or on-line process.

7. The method according to any one of the claims 1 to 6, wherein the entity of interest is one or more of Total Organic Carbon (TOC), O₂, CO₂, pH, bioburden, or conductivity.

8. A plant for measuring an entity of interest in a stream of rinsing water, comprising
a washed container (1) comprising a pharmaceutical product, dairy product, food product, or beverage product on a surface thereof,
an inlet (2) for supply of a stream of water for rinsing at least a part of the surface of the washed container (1), comprising the pharmaceutical product, dairy product, food product, or beverage product,
an inlet (3) configured for supplying a stream of air to the washed container (1),
an outlet (5) of the container(1) configured for collecting a mixture of water and air,
**characterized in that** said plant comprises
a cyclone (7) for deaerating the mixture of water and air for producing an aqueous stream, and
a sensor (10) for measuring the concentration of an entity of interest in the aqueous stream.

9. The plant according to claim 8, wherein the plant further comprises a computer software program (11) configured for comparing the measured concentration of the entity of interest with a predetermined concentration of the entity of interest and determining that the washing process is validated if the measured concentration is at or below the predetermined concentration.

10. The plant according to any one of the claims 8 or 9, wherein, the washed container (1) comprises a pharmaceutical product on a surface thereof.

11. The plant according to any one of the claims 8 to 10, wherein a pump (6) is used for transporting the collected mixture of water and air from the outlet (5) of the washed container (1) to the cyclone (7).

12. The plant according to claim 11, wherein the pump (6) is a side channels pump or water ring pump.

13. The plant according to any one of the claims 8 to 12, wherein the cyclone (7) is a hydrocyclone.

14. The plant according to any one of the claims 8 to 13, wherein the sensor (10) is one or more of pH electrode, TOC sensor, bioburden sensor, oxygen sensor, CO₂ sensor, or conductivity sensor.

15. Use of the method according to any one of claims 1 to 7 for validating a washing process of a unit (1) previously used for accommodating a pharmaceutical product.

## Patentansprüche

1. Verfahren zum Messen einer Einheit von Interesse in einem Strom von Spülwasser, umfassend
a. Zuführen eines Stroms von Wasser zu einer Einheit (1), die zuvor einem Waschvorgang unterzogen wurde, um mindestens einen Teil der Flächen der gewaschenen Einheit (1) zu spülen,
b. Zuführen eines Stroms von Luft zu der gewaschenen Einheit (1),
c. Sammeln eines Gemischs aus Wasser und Luft an einem Auslass (5) der Einheit (1),
**dadurch gekennzeichnet, dass** das Verfahren umfasst:
d. Entlüften des Gemischs aus Wasser und Luft unter Verwendung eines Zyklons (7), um einen wässrigen Strom zu erzeugen, und
e. Messen der Konzentration einer Einheit von Interesse in dem wässrigen Strom.

2. Verfahren nach Anspruch 1, ferner umfassend die Schritte zum
f. Vergleichen der gemessenen Konzentration der Einheit von Interesse mit einer vorbestimmten Konzentration und
g. Bestimmen, dass der Waschvorgang validiert ist, wenn die gemessene Konzentration kleiner oder gleich der vorbestimmten Konzentration ist.

3. Verfahren nach Anspruch 2, wobei die Schritte a bis f wiederholt werden, wenn die gemessene Konzentration größer als die vorbestimmte Konzentration ist.

4. Verfahren nach einem der Ansprüche 1 bis 3, wobei das Gemisch aus Wasser und Luft kontinuierlich entlüftet wird.

5. Verfahren nach einem der Ansprüche 1 bis 4, wobei die Konzentration der Einheit von Interesse kontinuierlich gemessen wird.

6. Verfahren nach einem der Ansprüche 1 bis 5, wobei das Messen der Konzentration der Einheit von Interesse in dem wässrigen Strom kontinuierlich als ein Inline- oder Online-Prozess durchgeführt wird.

7. Verfahren nach einem der Ansprüche 1 bis 6, wobei die Einheit von Interesse gesamter organischer Kohlenstoff (TOC für *Total Organic Carbon*) und/oder O₂ und/oder CO₂ und/oder pH und/oder Keimbelastung und/oder Leitfähigkeit ist.

8. Anlage zum Messen einer Einheit von Interesse in einem Strom von Spülwasser, umfassend
einen gewaschenen Behälter (1), der ein pharmazeutisches Produkt, Milchprodukt, Nahrungsmittelprodukt oder Getränkeprodukt auf einer Fläche davon umfasst,
einen Einlass (2) zur Zufuhr eines Stroms von Wasser zum Spülen mindestens eines Teils der Fläche des gewaschenen Behälters (1), der das pharmazeutische Produkt, Milchprodukt, Nahrungsmittelprodukt oder Getränkeprodukt umfasst,
einen Einlass (3), der zum Zuführen eines Stroms von Luft zu dem gewaschenen Behälter (1) eingerichtet ist,
einen Auslass (5) des Behälters (1), der zum Sammeln eines Gemischs aus Wasser und Luft eingerichtet ist,
**dadurch gekennzeichnet, dass** die Anlage umfasst:
einen Zyklon (7) zum Entlüften des Gemischs aus Wasser und Luft, um einen wässrigen Strom zu erzeugen, und
einen Sensor (10) zum Messen der Konzentration einer Einheit von Interesse in dem wässrigen Strom.

9. Anlage nach Anspruch 8, wobei die Anlage ferner ein Computersoftwareprogramm (11) umfasst, das zum Vergleichen der gemessenen Konzentration der Einheit von Interesse mit einer vorbestimmten Konzentration der Einheit von Interesse und Bestimmen, dass der Waschvorgang validiert ist, wenn die gemessene Konzentration kleiner oder gleich der vorbestimmten Konzentration ist, eingerichtet ist.

10. Anlage nach einem der Ansprüche 8 oder 9, wobei der gewaschene Behälter (1) ein pharmazeutisches Produkt auf einer Fläche davon umfasst.

11. Anlage nach einem der Ansprüche 8 bis 10, wobei eine Pumpe (6) zum Transportieren des gesammelten Gemischs aus Wasser und Luft von dem Auslass (5) des gewaschenen Behälters (1) zu dem Zyklon (7) verwendet wird.

12. Anlage nach Anspruch 11, wobei die Pumpe (6) eine Seitenkanalpumpe oder eine Wasserringpumpe ist.

13. Anlage nach einem der Ansprüche 8 bis 12, wobei der Zyklon (7) ein Hydrozyklon ist.

14. Anlage nach einem der Ansprüche 8 bis 13, wobei der Sensor (10) eine pH-Elektrode und/oder ein TOC-Sensor und/oder ein Keimbelastungssensor und/oder ein Sauerstoffsensor und/oder ein CO₂-Sensor und/oder ein Leitfähigkeitssensor ist.

15. Verwendung des Verfahrens nach einem der Ansprüche 1 bis 7 zum Validieren eines Waschvorgangs einer Einheit (1), die zuvor zum Aufnehmen eines pharmazeutischen Produkts verwendet wurde.

## Revendications

1. Procédé de mesure d'une entité d'intérêt dans un flux d'eau de rinçage, comprenant
a. l'alimentation d'un flux d'eau vers une unité (1) précédemment soumise à un processus de lavage pour le rinçage d'une partie au moins des surfaces de l'unité (1) lavée,
b. l'alimentation d'un flux d'air vers l'unité (1) lavée,
c. la collecte d'une mélange d'eau et d'air au niveau d'une sortie (5) de l'unité (1),
**caractérisé en ce que** ledit procédé comprend
d. la désaération du mélange d'eau et d'air à l'aide d'un cyclone (7) pour produire un flux aqueux, et
e. la mesure de la concentration d'une entité d'intérêt dans le flux aqueux.

2. Procédé selon la revendication 1, comprenant en outre les étapes suivantes
f. comparaison de la concentration de l'entité d'intérêt avec une concentration prédéterminée et
g. détermination que le processus de lavage est validé si la concentration mesurée est égale ou inférieure à la concentration prédéterminée.

3. Procédé la revendication 2, dans lequel les étapes a à f sont répétées si la concentration mesurée est supérieure à la concentration prédéterminée.

4. Procédé selon l'une quelconque des revendications 1 à 3, dans lequel le mélange d'eau et d'air est désaéré en continu.

5. Procédé selon l'une quelconque des revendications 1 à 4, dans lequel la concentration de l'entité d'intérêt est mesurée en continu.

6. Procédé selon l'une quelconque des revendications 1 à 5, dans lequel la mesure de la concentration d'une entité d'intérêt dans le flux aqueux est effectuée en continu comme un processus en ligne ou sur ligne.

7. Procédé selon l'une quelconque des revendications 1 à 6, dans lequel l'entité d'intérêt est l'une ou plusieurs parmi le carbone organique total (COT), l'O₂, le CO₂, le pH, la charge microbienne, ou la conductivité.

8. Installation pour la mesure d'une entité d'intérêt dans un flux d'eau de rinçage, comprenant
un récipient lave (1) comprenant un produit pharmaceutique, un produit laitier, un produit alimentaire, ou une boisson sur la surface de celui-ci,
une entrée (2) pour l'alimentation d'un flux d'eau destiné à rincer une partie au moins de la surface du récipient lave (1), comprenant le produit pharmaceutique, le produit laitier, le produit alimentaire, ou la boisson,
une entrée (3) configurée pour alimenter un flux d'air vers le récipient lave (1),
une sortie (5) du récipient (1) configurée pour collecter un mélange d'eau et d'air,
**caractérisée en ce que** ladite installation comprend
un cyclone (7) pour la désaération du mélange d'eau et d'air pour la production d'un flux aqueux, et
un capteur (10) pour la mesure de la concentration d'une entité d'intérêt dans le flux aqueux.

9. Installation selon la revendication 8, dans laquelle l'installation comprend en outre un programme logiciel informatique (11) configuré pour comparer la concentration mesurée de l'entité d'intérêt avec une concentration prédéterminée de l'entité d'intérêt et pour déterminer que le processus de lavage est validé si la concentration mesurée est égale ou inférieure à la concentration prédéterminée.

10. Installation selon l'une quelconque des revendications 8 ou 9, dans laquelle le récipient lave (1) comprend un produit pharmaceutique sur une surface de celui-ci.

11. Installation selon l'une quelconque des revendications 8 à 10, dans lequel une pompe (6) est utilisée pour transporter le mélange d'eau et d'air collecté à partir de la sortie (5) du récipient lave (1) vers le cyclone (7).

12. Installation selon la revendication 11, dans laquelle la pompe (6) est une pompe à canaux latéraux ou une pompe à anneau d'eau.

13. Installation selon l'une quelconque des revendications 8 à 12, dans laquelle le cyclone (7) est un hydrocyclone.

14. Installation selon l'une quelconque des revendications 8 à 13, dans laquelle le capteur (10) est l'un ou plusieurs parmi une électrode de pH, un capteur de COT, un capteur de charge microbienne, un capteur d'oxygène, un capteur de CO₂, ou un capteur de conductivité.

15. Utilisation du procédé selon l'une quelconque des revendications 1 à 7 pour la validation d'un processus de lavage d'une unité (1) précédemment utilisée pour accueillir un produit pharmaceutique.
